# EUROPEAN PATENT APPLICATION

(11) **EP 3 932 469 A1**
(43) Date of publication of application: **05.01.2022**
(21) Application number: 20777931.5
(22) Date of filing: 06.02.2020
(51) Int. Cl.: A61M 39/28, A61M 1/02

(54) **BLOOD BAG SYSTEM AND CLAMP**

(30) Priority: 27.03.2019 JP 2019060681
(71) Applicant: TERUMO Kabushiki Kaisha, Tokyo 151-0072 (JP)
(72) Inventor: OHASHI, Hirotaka, Tokyo 163-1450 (JP); NAKANO, Masanori, Fujinomiya-shi, Shizuoka 418-0004 (JP); SUZUKI, Takayuki, Tokyo 163-1450 (JP)
(74) Representative: TBK
(86) International application number: PCT/JP2020/004486
(87) International publication number: WO 2020/195207

(57) **Abstract**

A clamp (43) of a blood bag system (10) has a first clamp mechanism (102) for closing a second flow path (36a) of a second tube (36), and a second clamp mechanism (104) for closing a third flow path (38a) of a third tube (38). The first clamp mechanism (102) is configured not to open the second flow path (36a) of the second tube (36) once closed, and the second clamp mechanism (104) is configured to open the third flow path (38a) of the closed third tube (38) .

## Description

### BLOOD BAG SYSTEM AND CLAMP

### Technical Field

The present invention relates to a blood bag system and a clamp.

### Background Art

For example, JP 2016-106012 A discloses a blood bag system including a first bag (parent bag) accommodating blood, a second bag (child bag) accommodating a blood component obtained by centrifuging the blood in the first bag, and a third bag (medical solution bag) accommodating an additive solution.

A first tube connected to the first bag is coupled, via a branch, to a second tube connected to the second bag and a third tube connected to the third bag.

### Summary of Invention

In the blood bag system described above, the blood bag system is set in a centrifugation transfer apparatus, and the blood in the first bag is centrifuged. Then, the blood component obtained by centrifugation is transferred from the first bag to the second bag via the first tube and the second tube. Thereafter, the blood bag system is removed from the centrifugation transfer apparatus in a state where a flow path of the second tube is closed by a first clamp and a flow path of the third tube is closed by a second clamp. Subsequently, the third bag is hung on a suspension stand, the second clamp is removed from the third tube, and the additive solution such as a red blood cell preservation solution is transferred to the first bag via the third tube and the first tube.

When the first clamp for closing the flow path of the second tube and the second clamp for closing the flow path of the third tube are separate parts in this way, the flow paths of the two tubes (the second tube and the third tube) may not be efficiently closed. Further, there is a possibility that the second clamp is erroneously removed when the additive solution is transferred.

The present invention has been made in view of such problems, and an object of the invention is to provide a blood bag system and a clamp that can efficiently close a flow path of a second tube and a flow path of a third tube and can prevent the flow path of the second tube from being erroneously opened when an additive solution is transferred.

One aspect of the present invention is a blood bag system including a first bag configured to accommodate blood, a second bag configured to accommodate a blood component obtained by centrifuging the blood in the first bag, and a third bag configured to accommodate an additive solution, in which a first tube connected to the first bag is coupled, via a branch, to a second tube connected to the second bag and a third tube connected to the third bag, the branch is provided with one clamp, the clamp includes a first clamp mechanism configured to close a flow path of the second tube and a second clamp mechanism configured to close a flow path of the third tube, the first clamp mechanism is configured not to open the flow path of the second tube that has been closed once, and the second clamp mechanism is configured to open the flow path of the third tube that has been closed.

Another aspect of the present invention is a clamp including one clamp provided in a branch in a blood bag system, in which the blood bag system includes a first bag configured to accommodate blood, a second bag configured to accommodate a blood component obtained by centrifuging the blood in the first bag, a third bag configured to accommodate an additive solution, a first tube connecting the first bag and the branch, a second tube connecting the branch and the second bag, and a third tube connecting the branch and the third bag, the clamp includes a first clamp mechanism configured to close a flow path of the second tube, and a second clamp mechanism configured to close a flow path of the third tube, the first clamp mechanism is configured not to open the flow path of the second tube that has been closed once, and the second clamp mechanism is configured to open the flow path of the third tube that has been closed.

Still another aspect of the present invention is a blood bag system including a first bag configured to accommodate blood, a second bag configured to accommodate a blood component obtained by centrifuging the blood in the first bag, and a third bag configured to accommodate an additive solution, in which a first tube connected to the first bag is coupled, via a branch, to a second tube connected to the second bag and a third tube connected to the third bag, the branch is provided with one clamp, the clamp includes a first clamp mechanism and a second clamp mechanism, the first clamp mechanism includes a first arm having flexibility, a first protrusion provided on the first arm and pressing an outer peripheral surface of the second tube to close a flow path of the second tube, and a first engaging portion engaging with the first arm in a state where the flow path of the second tube is closed, and the second clamp mechanism includes a second arm having flexibility, a second protrusion provided on the second arm and pressing an outer peripheral surface of the third tube to close a flow path of the third tube, a second engaging portion engaging with the second arm in a state where the flow path of the third tube is closed, and an engagement releaser releasing engagement between the second engaging portion and the second arm.

In the present invention, the flow paths of the second tube and the third tube can be efficiently closed by one clamp including the first clamp mechanism and the second clamp mechanism. Further, the first clamp mechanism is configured not to open the flow path of the second tube once closed, and the second clamp mechanism is configured to close the closed flow path of the third tube.
Therefore, in the blood bag system, when the additive solution in the third bag is transferred to the first bag, only the flow path of the third tube is opened while the flow path of the second tube is prevented from being erroneously opened.

### Brief Description of Drawings

Fig. 1 is an explanatory view illustrating an overall configuration of a blood bag system and a centrifugation transfer apparatus according to an embodiment of the present invention.
Fig. 2 is a plan view illustrating a unit set area of the centrifugation transfer apparatus.
Fig. 3 is an explanatory plan view of a clamp in Fig. 1.
Fig. 4A is a first explanatory sectional view of the clamp in Fig. 3, and Fig. 4B is a second explanatory sectional view of the clamp in Fig. 3.
Fig. 5 is an explanatory view of a procedure for transferring a red blood cell preservation solution from a medical solution bag to a blood bag.
Fig. 6 is an explanatory sectional view of a clamp in Fig. 5.

### Description of Embodiments

Hereinafter, a preferred embodiment of a blood bag system and a clamp of the present invention will be described with reference to the accompanying drawings.

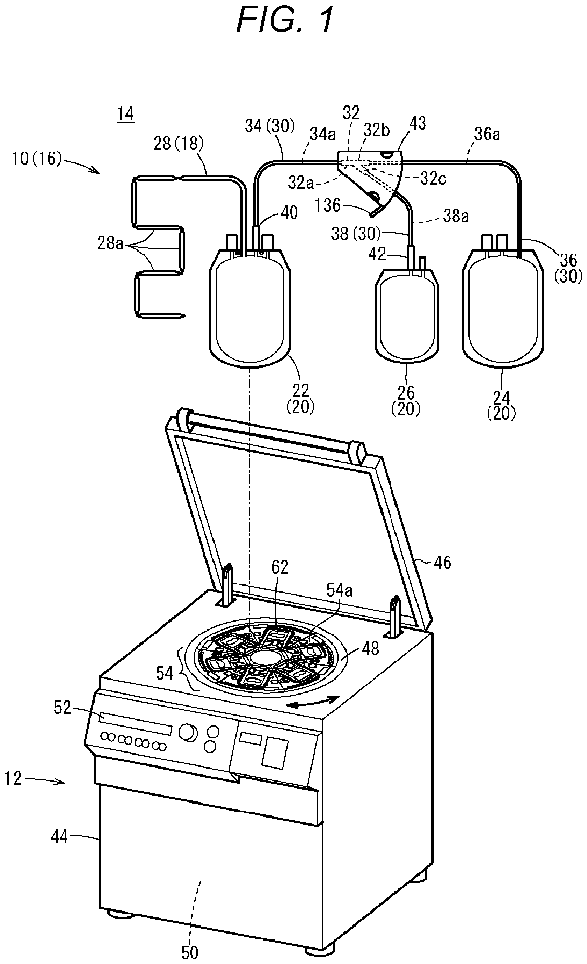

A blood bag system 10 according to an embodiment of the present invention is set in a centrifugation transfer apparatus 12 as illustrated in Fig. 1 by a user such as a medical worker. The centrifugation transfer apparatus 12 centrifuges blood in the blood bag system 10 to generate and store a plurality of types of blood components. A configuration including the blood bag system 10 and the centrifugation transfer apparatus 12 is referred to as a blood product manufacturing system 14.

The blood bag system 10 includes a pre-processor (not illustrated) used for blood collection before centrifugation, and a separation processor 16 that generates a blood component by centrifugation and individually stores the blood component, and the preprocessor and the separation processor are connected by a relay tube 18. The relay tube 18 connected to the preprocessor is cut before centrifugation, and the separation processor 16 is in a state shown in Fig. 1. Then, the separation processor 16 is set in the centrifugation transfer apparatus 12.

The preprocessor of the blood bag system 10 collects whole blood from a donor and removes a predetermined blood component (for example, white blood cells) from the whole blood. Thus, the preprocessor includes a blood sampling needle, a blood sampling bag, an initial flow blood bag, a filter (for example, a white blood cell removal filter), and the like, and is configured by connecting each member by a plurality of tubes. The separation processor 16 is connected to downstream of the filter via the relay tube 18. Specifically, the processor stores initial blood of the whole blood of the donor collected through the blood collection needle in the initial flow blood bag, and then stores residual blood in the blood collection bag. Furthermore, the processor removes white blood cells in the filter by causing the whole blood stored in the blood collection bag to flow to the filter, and transfers the removed blood (white blood cell removed blood) to the separation processor 16.

The separation processor 16 of the blood bag system 10 includes a plurality of bags 20 (a blood bag 22, a PPP bag 24, and a medical solution bag 26), and is configured by connecting the bags 20 by a plurality of tubes 30.

The blood bag 22 (first bag) is directly connected to the relay tube 18 connected to the pre-processor in a state of the blood bag system 10 provided as a product. Thus, the blood bag 22 stores the removed blood transferred from the filter during blood collection. The blood bag 22 is set in the centrifugation transfer apparatus 12, and a centrifugal force is applied by operation of the centrifugation transfer apparatus 12. As a result, the removed blood in the blood bag 22 is centrifuged into blood components (platelet poor plasma (PPP), red blood cells (RBC)), and the like having different specific gravities. Then, the blood bag 22 stores only the residual RBCs by transferring PPP during the operation of the centrifugation transfer apparatus 12 after centrifugation.

The PPP is supplied from the blood bag 22, and the PPP bag 24 (second bag) stores the PPP. On the other hand, the medical solution bag 26 (third bag) stores in advance a red blood cell preservation solution (additive solution) such as MAP solution, SAGM solution, or OPTISOL.

Further, a segment tube 28 is formed in the separation processor 16 before the separation processor 16 is set in the centrifugation transfer apparatus 12. The segment tube 28 has a plurality of sealed tubes 28a in which blood of a donor (removed blood) exists. The relay tube 18 connecting the blood bag 22 and the pre-processor (filter) is cut near the filter and is further sealed at predetermined intervals, and thus the plurality of sealed tubes 28a is formed so as to be continuous in series. The segment tubes 28 are cut by the user as necessary and used for checking blood and the like.

The plurality of tubes 30 of the separation processor 16 is branched into a plurality of tubes via a branch connector 32 (Y-shaped connector) as a branch. Specifically, the plurality of tubes 30 includes a first tube 34, a second tube 36, and a third tube 38. The first tube 34 connects the blood bag 22 and a first connector portion 32a of the branch connector 32. The second tube 36 connects the PPP bag 24 and the second connector portion 32b of the branch connector 32. The third tube 38 connects the medical solution bag 26 and the third connector portion 32c of the branch connector 32.

The first tube 34 has a first flow path 34a, the second tube 36 has a second flow path 36a, and the third tube 38 has a third flow path 38a. The first tube 34, the second tube 36, and the third tube 38 have flexibility. The first flow path 34a, the second flow path 36a, and the third flow path 38a communicate with each other via a flow path in the branch connector 32.

A breakable sealing member 40 (click tip) is provided at an end of the first tube 34 closer to the blood bag 22. Similarly, a breakable sealing member 42 is provided at an end of the third tube 38 closer to the medical solution bag 26. The sealing members 40 and 42 block the first flow path 34a and the third flow path 38a until breaking operation is performed, and prevent a liquid in the blood bag 22 and the medical solution bag 26 from being transferred to another bag 20.

The separation processor 16 includes a clamp 43 provided in the branch connector 32. A configuration of the clamp 43 will be described later.

The centrifugation transfer apparatus 12 in which the separation processor 16 of the blood bag system 10 is set includes a box-shaped base body 44, a lid 46 capable of opening and closing an upper surface of the base body 44, and a centrifugal drum 48 provided in the base body 44. Further, the base body 44 of the centrifugation transfer apparatus 12 is provided with a motor (not illustrated) that rotates the centrifugal drum 48, a controller 50 that controls the operation of the centrifugation transfer apparatus 12, and an operation display 52 for the user to confirm and operate.

The centrifugal drum 48 has a plurality of (six) unit set areas 54 in which the separation processor 16 can be set. A height of one unit set area 54 is longer than a longitudinal length of the bag 20, and is set in a range of 60° with respect to a rotation center of the centrifugal drum 48. That is, the six unit set areas 54 are arranged without a gap along a circumferential direction to constitute the centrifugal drum 48 as an annular structure.

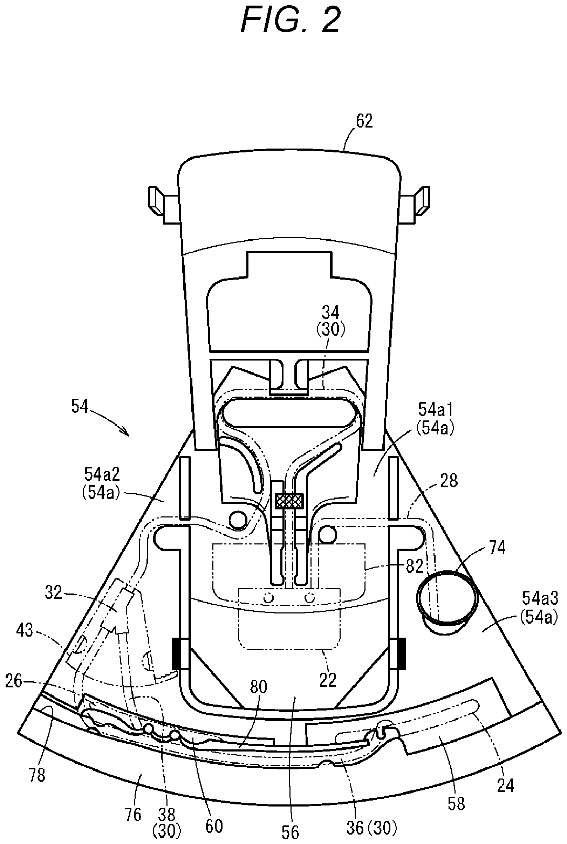

The unit set areas 54 apply a centrifugal force to the blood bag 22 as the centrifugal drum 48 rotates. Specifically, as illustrated in Fig. 2, each of the unit set areas 54 includes a blood bag pocket 56 accommodating the blood bag 22, a PPP bag pocket 58 accommodating the PPP bag 24, and a medical solution bag pocket 60 accommodating the medical solution bag 26 radially outside of the centrifugal drum 48.

The blood bag pocket 56 is provided at a circumferential center of the unit set area 54 and has a larger volume than the PPP bag pocket 58 and the medical solution bag pocket 60. The PPP bag pocket 58 and the medical solution bag pocket 60 are provided side by side in the circumferential direction radially outside of the blood bag pocket 56.

An upper surface 54a of the unit set area 54 is configured to arrange and hold the plurality of tubes 30 of the blood bag system 10. A part of the first tube 34 and a part of the segment tube 28 are disposed in a central region 54a1 (first region) radially inside of the blood bag pocket 56 in the upper surface 54a.

The central region 54a1 is provided with a lid 62 that opens and closes an opening of the blood bag pocket 56. A part of the first tube 34, a part of the clamp 43, a part of the second tube 36, and a part of the third tube 38 that have passed through the central region 54a1 are disposed in a left region 54a2 (second region) of the upper surface 54a. Note that the left region 54a2 may be provided with a holder (not illustrated) that holds the clamp 43.

A part of the segment tube 28 that passes through the central region 54a1 is disposed in a right region 54a3 (third region) of the upper surface 54a. The right region 54a3 is provided with a segment pocket 74 that accommodates the plurality of sealed tubes 28a of the segment tube 28.

Furthermore, a tube holder 76 is provided radially outside of the PPP bag pocket 58 and the medical solution bag pocket 60 of the unit set area 54. The tube holder 76 has an outer wall 78 continuous with an outer peripheral edge of the unit set area 54 and an inner wall 80 protruding inward from the outer peripheral edge with respect to the outer wall 78, and the second tube 36 is disposed between the outer wall 78 and the inner wall 80.

Further, the unit set area 54 includes a slider 82 (pusher) that presses the blood bag 22 after centrifugation radially inside of the blood bag pocket 56. The slider 82 moves forward and backward along a radial direction of the centrifugal drum 48 under the control of the controller 50 (see Fig. 1).

In the unit set area 54, the user accommodates the blood bag 22 storing the removed blood in the blood bag pocket 56, the empty PPP bag 24 in the PPP bag pocket 58, and the medical solution bag 26 storing the red blood cell preservation solution in the medical solution bag pocket 60. Then, the unit set area 54 centrifuges the blood removed from the blood bag 22 by the rotation of the centrifugal drum 48, advances the slider 82 after the centrifugation, and presses the blood bag 22.

As a result, PPP generated by centrifugation in the blood bag 22 is transferred to the PPP bag 24, and the RBC remains in the blood bag 22 after the transfer of PPP. Thereafter, the blood bag system 10 is taken out from the centrifugation transfer apparatus 12 by the user, and the medical solution bag 26 is hung on a stand (not illustrated). As a result, the red blood cell preservation solution is supplied from the medical solution bag 26 to the blood bag 22, and the blood bag 22 is storing RBC (blood product) including a medical solution.

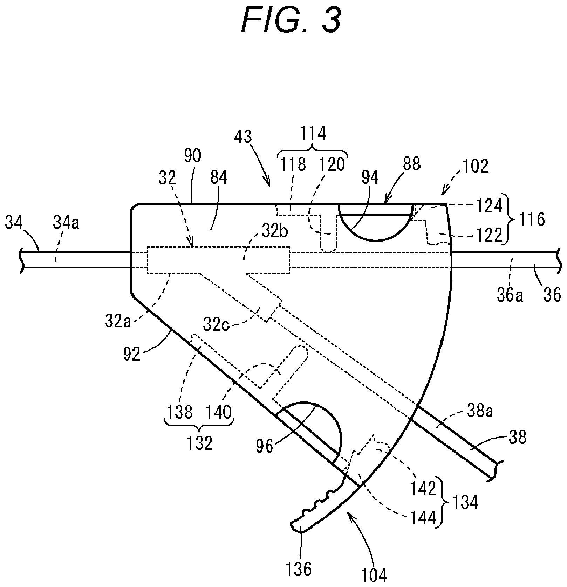

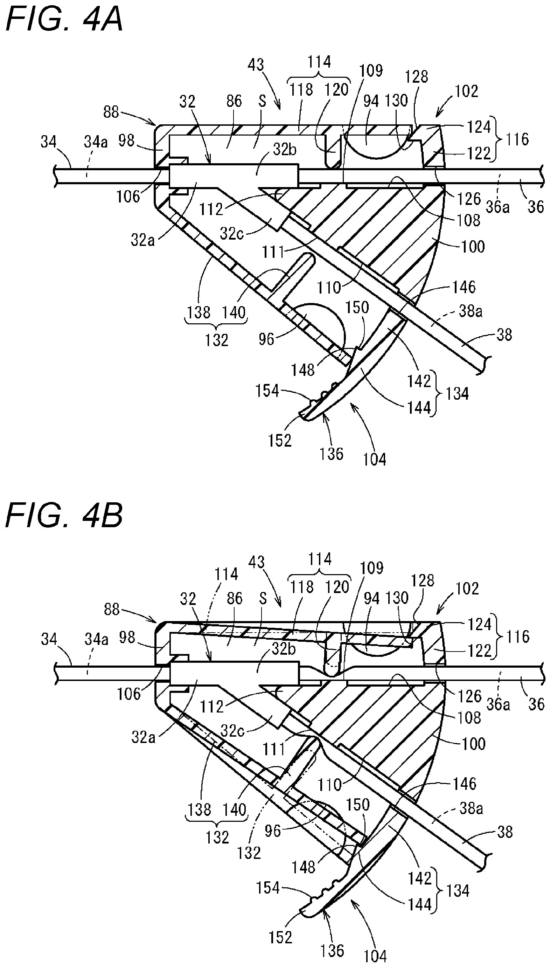

As illustrated in Figs. 3 and 4A, the clamp 43 of the blood bag system 10 has an accommodation space S that accommodates the branch connector 32, a part of the second tube 36, and a part of the third tube 38. The clamp 43 includes a first cover 84 and a second cover 86 disposed to face each other, and a clamp body 88 provided to connect the first cover 84 and the second cover 86 to each other. The first cover 84 and the second cover 86 are disposed to sandwich the branch connector 32.

In Fig. 3, the first cover 84 has a flat plate shape, and has a width increasing from one end where the first connector portion 32a is located toward the other end where the second tube 36 and the third tube 38 are located. The first cover 84 includes a first side 90 and a second side 92. The first side 90 extends from one end to the other end of the first cover 84 along an extending direction of the second connector portion 32b. The second side 92 extends from one end to the other end of the first cover 84 along an extending direction of the third connector portion 32c.

The first cover 84 is provided with a first cutout 94 and a second cutout 96. The first cutout 94 extends in a semicircular shape from the first side 90 to a side on which the second tube 36 is located. The first cutout 94 is shifted toward the other end of the first cover 84 from a center of the first side 90.

The second cutout 96 extends in a semicircular shape from the second side 92 to a side on which the third tube 38 is located. The second cutout 96 is shifted toward the other end of the first cover 84 from a center of the second side 92. Each of the first cutout 94 and the second cutout 96 is set to a size that allows insertion of the user's finger. However, a shape and position of each of the first cutout 94 and the second cutout 96 can be appropriately changed.

The second cover 86 is configured similarly to the first cover 84. Therefore, description of the configuration of the second cover 86 is omitted.

In Fig. 4A, the clamp body 88 is integrally molded by a flexible resin material. The clamp body 88 includes a first wall 98 disposed closer to the first tube 34, a second wall 100 disposed between the second tube 36 and the third tube 38, a first clamp mechanism 102 closing the second flow path 36a of the second tube 36, and a second clamp mechanism 104 closing the third flow path 38a of the third tube 38.

The first wall 98 is provided with a first insertion hole 106 through which the first tube 34 is inserted. The first wall 98 supports the first connector portion 32a of the branch connector 32. The first wall 98 is coupled to the first cover 84 and the second cover 86.

The second wall 100 is coupled to the first cover 84 and the second cover 86. The second wall 100 has a triangular shape when viewed from a thickness direction of the clamp 43 (a direction in which the first cover 84 and the second cover 86 are aligned). The second wall 100 has a first side surface 108 extending along the second tube 36 and a second side surface 110 extending along the third tube 38.

The first side surface 108 is in contact with or in proximity to an outer peripheral surface of the second connector portion 32b of the branch connector 32. The second side surface 110 is in contact with or in proximity to an outer peripheral surface of the third connector portion 32c of the branch connector 32. A corner 112 formed by the first side surface 108 and the second side surface 110 is located between the second connector portion 32b and the third connector portion 32c. On the first side surface 108, a first projection 109 that is in contact with an outer peripheral surface of the second tube 36 is formed. On the second side surface 110, a second projection 111 that is in contact with an outer peripheral surface of the third tube 38 is formed.

The first clamp mechanism 102 includes a first presser 114 pressing the outer peripheral surface of the second tube 36 so as to close the second flow path 36a of the second tube 36, and a first lock 116 locking the first presser 114.

The first presser 114 has flexibility. The first presser 114 is displaceable from a first open position (position in Fig. 4A) where the second flow path 36a of the second tube 36 is opened to a first closed position (position in Fig. 4B) where the second flow path 36a of the second tube 36 is closed. The first presser 114 includes a first arm 118 extending from the first wall 98 along the second tube 36 (the second connector portion 32b) and a first protrusion 120 protruding from an inner surface of the first arm 118.

The first arm 118 is located on an opposite side to the second wall 100 across the second tube 36. That is, the second tube 36 is disposed between the first side surface 108 (first projection 109) of the second wall 100 and the first arm 118. The first protrusion 120 is located at a position on the inner surface of the first arm 118 facing the second tube 36. The first presser 114 is not directly coupled to the first cover 84 and the second cover 86. Therefore, the first presser 114 is elastically deformable in a direction intersecting an extending direction of the first arm 118.

The first lock 116 locks the first presser 114 at the first closed position. Specifically, the first lock 116 includes a first extension 122 extending from the second wall 100 to a side where the first arm 118 is located, and a first claw 124 provided at an extending end of the first extension 122. The first extension 122 is provided with a second insertion hole 126 through which the second tube 36 is inserted. The first claw 124 closes the second flow path 36a of the second tube 36 by engaging with an extending end of the first arm 118. The first lock 116 is not directly coupled to the first cover 84 and the second cover 86. Therefore, the first lock 116 is elastically deformable in a direction intersecting the extending direction of the first extension 122.

The first claw 124 is provided with a first inclined surface 128 and a first stopper surface 130 (first engaging portion). The first inclined surface 128 is inclined toward the branch connector 32 from a distal end of the first claw 124 toward a proximal end direction (a side on which the second wall 100 is located). When the first presser 114 is at the first open position, the extending end of the first arm 118 comes into contact with the first inclined surface 128 (see Fig. 4A). The first stopper surface 130 extends from an end of the first inclined surface 128 on a side on which the second wall 100 is located to an opposite side to the branch connector 32. When the first presser 114 is at the first closed position, an outer surface of the extending end of the first arm 118 comes into contact with the first stopper surface 130 (see

Fig. 4B).

The first clamp mechanism 102 does not have a function of unlocking the first lock 116. Thus, the first presser 114 is unreturnable from the first closed position to the first open position. In other words, the first clamp mechanism 102 is configured not to open the closed flow path of the second tube 36.

The second clamp mechanism 104 includes a second presser 132 pressing the outer peripheral surface of the third tube 38 so as to close the third flow path 38a of the third tube 38, a second lock 134 locking the second presser 132, and a lock releaser 136 unlocking the second lock 134.

The second presser 132 has flexibility. The second presser 132 is displaceable from a second open position (position in Fig. 4A) where the third flow path 38a of the third tube 38 is opened to a second closed position (position in Fig. 4B) where the third flow path 38a of the third tube 38 is closed. The second presser 132 has a second arm 138 extending from the first wall 98 along the third tube 38 (the third connector portion 32c) and a second protrusion 140 protruding from an inner surface of the second arm 138.

The second arm 138 is located on an opposite side to the second wall 100 across the third tube 38. That is, the third tube 38 is disposed between the second side surface 110 (the second projection 111) of the second wall 100 and the second arm 138. The second protrusion 140 is located at a position on the inner surface of the second arm 138 facing the third tube 38. The second presser 132 is not directly coupled to the first cover 84 and the second cover 86. Therefore, the second presser 132 is elastically deformable in a direction intersecting an extending direction of the second arm 138.

The second lock 134 locks the second presser 132 at the second closed position. Specifically, the second lock 134 includes a second extension 142 extending from the second wall 100 to a side where the second arm 138 is located, and a second claw 144 provided at an extending end of the second extension 142. The second extension 142 is provided with a third insertion hole 146 through which the third tube 38 is inserted. The second claw 144 closes the third flow path 38a of the third tube 38 by engaging with an extending end of the second arm 138. The second lock 134 is not directly coupled to the first cover 84 and the second cover 86. Therefore, the second lock 134 is elastically deformable in a direction intersecting an extending direction of the second extension 142.

The second claw 144 is provided with a second inclined surface 148 and a second stopper surface 150 (second engaging portion). The second inclined surface 148 is inclined toward the branch connector 32 from a distal end of the second claw 144 toward the proximal end direction (the side on which the second wall 100 is located). When the second presser 132 is at the second open position, the extending end of the second arm 138 comes into contact with the second inclined surface 148 (see Fig. 4A). The second stopper surface 150 extends from an end of the second inclined surface 148 on a side on which the second wall 100 is located to an opposite side to the branch connector 32. The second stopper surface 150 is in contact with the outer surface of the extending end of the second arm 138 when the second presser 132 is at the second closed position (see Fig. 4B).

The lock releaser 136 is an engagement releaser that releases engagement between the second stopper surface 150 and the extending end of the second arm 138. The lock releaser 136 includes an operating portion 152 extending from the second claw 144 to an opposite side to the second wall 100, and a plurality of nonslip protrusions 154 provided on the operating portion 152. The operating portion 152 has a size that allows a user to easily operate the operating portion with fingers. The operating portion 152 protrudes from the second claw 144 (wall) provided with the second stopper surface 150 toward outside of the second arm 138. The plurality of protrusions 154 protrudes from a surface of the operating portion 152 on a side where the first wall 98 is located. The plurality of protrusions 154 is provided along an extending direction of the operating portion 152. The protrusions 154 extend linearly in a thickness direction of the clamp 43. The second clamp mechanism 104 is configured to open the closed third flow path 38a of the third tube 38.

The blood bag system 10 according to the present embodiment is basically configured as described above, and the operation thereof will be described below.

As described above, the blood bag system 10 stores the removed blood obtained by removing a predetermined component (white blood cells) from the whole blood of the donor in the blood bag 22 with use of the pre-processor during the blood collection by the user (medical worker). The blood bag 22 stores blood of an appropriate blood volume (for example, 400 cc) in accordance with the donor.

Thereafter, as illustrated in Fig. 2, in order to centrifuge the removed blood, the user separates the separation processor 16 of the blood bag system 10 from the pre-processor and sets the separation processor in the centrifugation transfer apparatus 12. The blood bag 22 is inserted into the blood bag pocket 56 of the unit set area 54. The first tube 34 is disposed in the central region 54a1 of the upper surface 54a. The medical solution bag 26 is inserted into the medical solution bag pocket 60, and the PPP bag 24 is inserted into the PPP bag pocket 58. The clamp 43 is disposed in the left region 54a2 of the upper surface 54a. When the user closes the lid 62, the sealing member 40 is broken to open the first flow path 34a of the first tube 34.

After the blood bag system 10 is set, the centrifugation transfer apparatus 12 centrifuges the blood removed from the blood bag 22 into blood components having different specific gravities (PPP, RBC, and the like) by rotating the centrifugal drum 48 under the control of the controller 50. After this centrifugation, the centrifugation transfer apparatus 12 presses the blood bag 22 by the slider 82. As a result, PPP having a light specific gravity flows out from the blood bag 22, and the PPP flows through the first tube 34, the branch connector 32, and the second tube 36 in that order and flows into the PPP bag 24.

When PPP is transferred from the blood bag 22 to the PPP bag 24, the centrifugation transfer apparatus 12 retracts the slider 82 such that the blood bag 22 can be taken out from the blood bag pocket 56. Then, the user closes the second flow path 36a of the second tube 36 and the third flow path 38a of the third tube 38 by the clamp 43.

That is, as illustrated in Fig. 4B, the user presses the outer surface of the first arm 118 of the clamp 43 inward (toward the side on which the second wall 100 is located). At this time, the user can easily press the first arm 118 by inserting the finger into the first cutout 94 of the first cover 84 and the second cover 86.

Then, the first arm 118 is bent (elastically deformed) toward the second wall 100, and thus the outer peripheral surface of the second tube 36 is pushed toward the second wall 100 by the first protrusion 120. In other words, the second tube 36 is sandwiched between the first projection 109 of the second wall 100 and the first protrusion 120. Thus, the second flow path 36a of the second tube 36 is closed. That is, the first presser 114 is displaced from the first open position to the first closed position. Further, the first lock 116 is pushed outward by the first arm 118 and is bent (elastically deformed), and thus the first arm 118 that has passed over the first inclined surface 128 comes into contact with the first stopper surface 130. As a result, the first presser 114 is locked at the first closed position.

The user presses the outer surface of the second arm 138 of the clamp 43 inward (toward the side on which the second wall 100 is located). At this time, the user can easily press the second arm 138 by inserting the finger into the second cutout 96 of the first cover 84 and the second cover 86.

Then, the second arm 138 is bent (elastically deformed) toward the second wall 100, and thus the outer peripheral surface of the third tube 38 is pushed toward the second wall 100 by the second protrusion 140. In other words, the third tube 38 is sandwiched between the second projection 111 of the second wall 100 and the second protrusion 140. Thus, the third flow path 38a of the third tube 38 is closed. That is, the second presser 132 is displaced from the second open position to the second closed position. Further, the second lock 134 is pushed outward by the second arm 138 and is bent (elastically deformed), and thus the second arm 138 that has passed over the second inclined surface 148 comes into contact with the second stopper surface 150. As a result, the second presser 132 is locked at the second closed position.

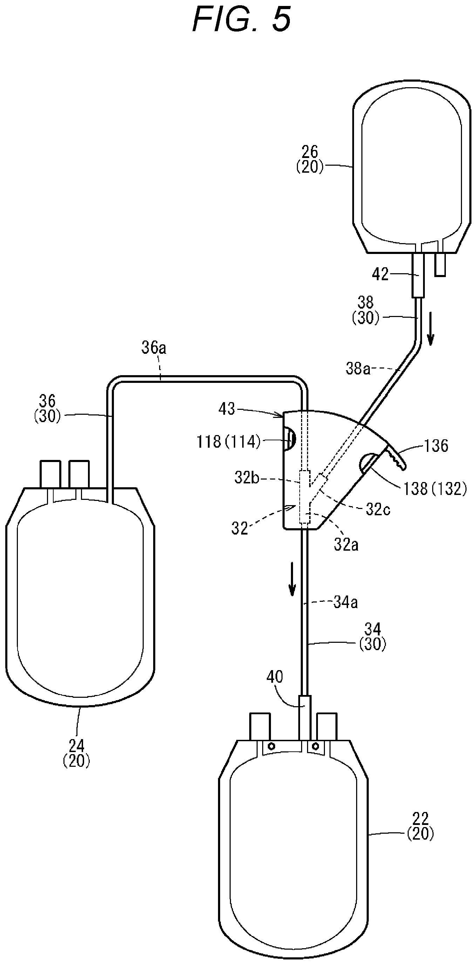

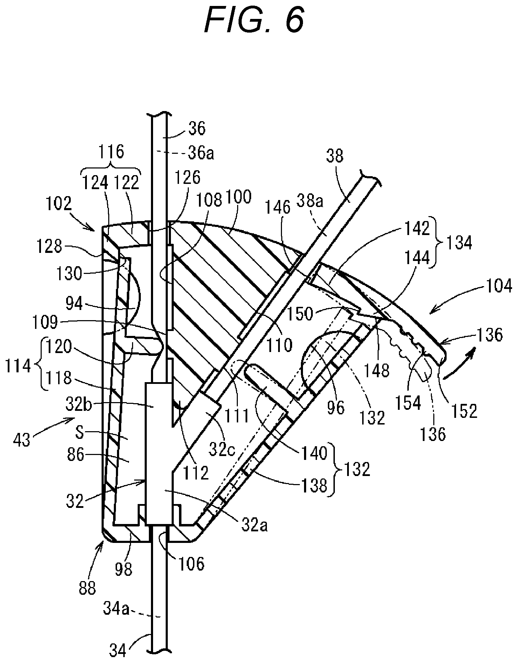

Thereafter, as illustrated in Fig. 5, the user takes out the blood bag system 10 from the centrifugation transfer apparatus 12, and suspends the medical solution bag 26 on a stand (not illustrated). Then, the third flow path 38a of the third tube 38 is opened. Specifically, in Fig. 6, the user operates the operating portion 152 of the lock releaser 136 in a direction away from the first wall 98. Then, the second extension 142 is bent (elastically deformed) to increase a distance between the first wall 98 and the second claw 144, and thus the second arm 138 is separated from the second stopper surface 150. As a result, the second arm 138 returns to an original shape by a restoring force, the second presser 132 returns from the second closed position to the second open position.

At this time, the first presser 114 remains at the first closed position. That is, the second flow path 36a of the second tube 36 is maintained closed. This prevents the red blood cell preservation solution from flowing into the PPP bag 24. As a result, the RBC including the red blood cell preservation solution is stored in the blood bag 22.

In this case, the blood bag system 10 and the clamp 43 according to the present embodiment have the following effects.

The clamp 43 includes the first clamp mechanism 102 closing the second flow path 36a of the second tube 36 and the second clamp mechanism 104 closing the third flow path 38a of the third tube 38. The first clamp mechanism 102 is configured not to open the second flow path 36a of the second tube 36 once closed, and the second clamp mechanism 104 is configured to open the closed third flow path 38a of the third tube 38.

In such a configuration, the second flow path 36a of the second tube 36 and the third flow path 38a of the third tube 38 can be efficiently closed by one clamp 43 including the first clamp mechanism 102 and the second clamp mechanism 104. Further, the first clamp mechanism 102 is configured not to open the second flow path 36a of the second tube 36 once closed, and the second clamp mechanism 104 is configured to close the closed third flow path 38a of the third tube 38. Therefore, in the blood bag system 10, when the red blood cell preservation solution in the medical solution bag 26 is transferred to the blood bag 22, only the third flow path 38a of the third tube 38 can be opened while the second flow path 36a of the second tube 36 is prevented from being erroneously opened.

The first clamp mechanism 102 includes the first presser 114 pressing the outer peripheral surface of the second tube 36 so as to close the second flow path 36a of the second tube 36. The second clamp mechanism 104 includes the second presser 132 pressing the outer peripheral surface of the third tube 38 so as to close the third flow path 38a of the third tube 38.

In such a configuration, the second flow path 36a of the second tube 36 can be reliably closed by the first presser 114. Further, the third flow path 38a of the third tube 38 can be reliably closed by the second presser 132.

The first presser 114 is displaceable from the first open position where the second flow path 36a of the second tube 36 is opened to the first closed position where the second flow path 36a of the second tube 36 is closed, and is unreturnable from the first closed position to the first open position. The second presser 132 is displaceable from the second open position where the third flow path 38a of the third tube 38 is opened to the second closed position where the third flow path 38a of the third tube 38 is closed, and is returnable from the second closed position to the second open position.

In such a configuration, the second flow path 36a of the second tube 36 can be easily closed by displacing the first presser 114 from the first open position to the first closed position. The first presser 114 is unreturnable from the first closed position to the first open position, and thus the second flow path 36a of the second tube 36 once closed is not opened. By displacing the second presser 132 from the second open position to the second closed position, the third flow path 38a of the third tube 38 can be easily closed. Further, by returning the second presser 132 from the second closed position to the second open position, the closed third flow path 38a of the third tube 38 can be easily opened.

The first clamp mechanism 102 includes the first lock 116 locking the first presser 114 at the first closed position. The second clamp mechanism 104 includes the second lock 134 locking the second presser 132 at the second closed position, and the lock releaser 136 unlocking the second lock 134 to return the second presser 132 located at the second closed position to the second open position.

In such a configuration, the first presser 114 can be held at the first closed position by the first lock 116, and the second presser 132 can be held at the second closed position by the second lock 134. Further, by unlocking the second lock 134 by the lock releaser 136, the second presser 132 can be returned from the second closed position to the second open position.

The clamp 43 includes the second wall 100 provided between the second tube 36 and the third tube 38. In a state where the first presser 114 is located at the first closed position, the second tube 36 is sandwiched between the second wall 100 and the first presser 114. In a state where the second presser 132 is located at the second closed position, the third tube 38 is sandwiched between the second wall 100 and the second presser 132.

In such a configuration, the second flow path 36a of the second tube 36 and the third flow path 38a of the third tube 38 can be easily closed.

The lock releaser 136 includes the operating portion 152 protruding from the second claw 144 provided with the second stopper surface 150 toward outside of the second arm 138.

In such a configuration, the user can easily operate the operating portion 152, and thus can easily release the engagement between the second stopper surface 150 and the second claw 144.

The present invention is not limited to the above embodiment, and various modifications can be made without departing from the gist of the present invention.

In the clamp of the present invention, the first clamp mechanism may be configured such that the first presser 114 is displaced from the first open position to the first closed position by a user's operation from a direction orthogonal to a plane passing through the second connector portion 32b and the third connector portion 32c. Further, the second clamp mechanism may be configured such that the second presser 132 is displaced from the second open position to the second closed position by a user's operation from a direction orthogonal to a plane passing through the second connector portion 32b and the third connector portion 32c. In this case, the user can operate the clamp from above in a state where the clamp is set in the left region 54a2 of the unit set area 54 of the centrifugation transfer apparatus 12, and thus operability of the clamp can be improved.

The above embodiment is summarized as follows.

The present embodiment discloses a blood bag system (10) including a first bag (22) configured to accommodate blood, a second bag (24) configured to accommodate a blood component obtained by centrifuging the blood in the first bag (22), and a third bag (26) configured to accommodate an additive solution, in which a first tube (34) connected to the first bag (22) is coupled, via a branch (32), to a second tube (36) connected to the second bag (24) and a third tube (38) connected to the third bag (26), the branch (32) is provided with one clamp (43), the clamp (43) includes a first clamp mechanism (102) configured to close a flow path (36a) of the second tube (36) and a second clamp mechanism (104) configured to close a flow path (38a) of the third tube (38), the first clamp mechanism (102) is configured not to open the flow path (36a) of the second tube (36) that has been closed once, and the second clamp mechanism (104) is configured to open the flow path (38a) of the third tube (38) that has been closed.

In the blood bag system (10), the first clamp mechanism (102) may include a first presser (114) pressing an outer peripheral surface of the second tube (36) to close the flow path (36a) of the second tube (36), and the second clamp mechanism (104) may include a second presser (132) pressing an outer peripheral surface of the third tube (38) to close the flow path (38a) of the third tube (38).

In the blood bag system (10), the first presser (114) may be displaceable from a first open position where the flow path (36a) of the second tube (36) is opened to a first closed position where the flow path (36a) of the second tube (36) is closed and may be unreturnable from the first closed position to the first open position, and the second presser (132) may be displaceable from a second open position where the flow path (38a) of the third tube (38) is opened to a second closed position where the flow path (38a) of the third tube (38) is closed and may be returnable from the second closed position to the second open position.

In the blood bag system (10), the first clamp mechanism (102) may include a first lock (116) locking the first presser (114) at the first closed position, and the second clamp mechanism (104) may include a second lock (134) locking the second presser (132) at the second closed position, and a lock releaser (136) unlocking the second lock (134) to return the second presser (132) located at the second closed position to the second open position.

In the blood bag system (10), the clamp (43) may include a wall (100) provided between the second tube (36) and the third tube (38), the second tube (36) may be sandwiched between the wall (100) and the first presser (114) in a state where the first presser (114) is located at the first closed position, and the third tube (38) may be sandwiched between the wall (100) and the second presser (132) in a state where the second presser (132) is located at the second closed position.

The present embodiment discloses a clamp (43) including one clamp (43) provided in a branch (32) in a blood bag system (10), in which the blood bag system (10) includes a first bag (22) configured to accommodate blood, a second bag (24) configured to accommodate a blood component obtained by centrifuging the blood in the first bag (22), a third bag (26) configured to accommodate an additive solution, a first tube (34) connecting the first bag (22) and the branch (32), a second tube (36) connecting the branch (32) and the second bag (24), and a third tube (38) connecting the branch (32) and the third bag (26), the clamp (43) includes a first clamp mechanism (102) configured to close a flow path (36a) of the second tube (36), and a second clamp mechanism (104) configured to close a flow path (38a) of the third tube (38), the first clamp mechanism (102) is configured not to open the flow path (36a) of the second tube (36) that has been closed once, and the second clamp mechanism (104) is configured to open the flow path (38a) of the third tube (38) that has been closed.

The present embodiment discloses a blood bag system (10) including a first bag (22) configured to accommodate blood, a second bag (24) configured to accommodate a blood component obtained by centrifuging the blood in the first bag (22), and a third bag (26) configured to accommodate an additive solution, in which a first tube (34) connected to the first bag (22) is coupled, via a branch (32), to a second tube (36) connected to the second bag (24) and a third tube (38) connected to the third bag (26), the branch (32) is provided with one clamp (43), the clamp (43) includes a first clamp mechanism (102) and a second clamp mechanism (104), the first clamp mechanism (102) includes a first arm (118) having flexibility, a first protrusion (120) provided on the first arm (118) and pressing an outer peripheral surface of the second tube (36) to close a flow path (36a) of the second tube (36), and a first engaging portion (130) engaging with the first arm (118) in a state where the flow path (36a) of the second tube (36) is closed, and the second clamp mechanism (104) includes a second arm (138) having flexibility, a second protrusion (140) provided on the second arm (138) and pressing an outer peripheral surface of the third tube (38) to close a flow path (38a) of the third tube (38), a second engaging portion (150) engaging with the second arm (138) in a state where the flow path (38a) of the third tube (38) is closed, and an engagement releaser (136) releasing engagement between the second engaging portion (150) and the second arm (138).

In the blood bag system (10), the engagement releaser (136) may include an operating portion (152) protruding from a wall (144) provided with the second engaging portion (150) toward outside of the second arm (138).

## Claims

1. A blood bag system comprising:
a first bag configured to accommodate blood; a second bag configured to accommodate a blood component obtained by centrifuging the blood in the first bag; and a third bag configured to accommodate an additive solution, wherein
a first tube connected to the first bag is coupled, via a branch, to a second tube connected to the second bag and a third tube connected to the third bag,
the branch is provided with one clamp,
the clamp includes
a first clamp mechanism configured to close a flow path of the second tube and
a second clamp mechanism configured to close a flow path of the third tube,
the first clamp mechanism is configured not to open the flow path of the second tube that has been closed once, and
the second clamp mechanism is configured to open the flow path of the third tube that has been closed.

2. The blood bag system according to claim 1, wherein
the first clamp mechanism includes a first presser that presses an outer peripheral surface of the second tube to close the flow path of the second tube, and
the second clamp mechanism includes a second presser that presses an outer peripheral surface of the third tube to close the flow path of the third tube.

3. The blood bag system according to claim 2, wherein
the first presser is displaceable from a first open position where the flow path of the second tube is opened to a first closed position where the flow path of the second tube is closed, and is unreturnable from the first closed position to the first open position, and
the second presser is displaceable from a second open position where the flow path of the third tube is opened to a second closed position where the flow path of the third tube is closed, and is returnable from the second closed position to the second open position.

4. The blood bag system according to claim 3, wherein
the first clamp mechanism includes a first lock locking the first presser at the first closed position, and
the second clamp mechanism includes
a second lock locking the second presser at the second closed position, and
a lock releaser unlocking the second lock to return the second presser located at the second closed position to the second open position.

5. The blood bag system according to claim 3 or 4, wherein
the clamp includes a wall provided between the second tube and the third tube,
the second tube is sandwiched between the wall and the first presser in a state where the first presser is located at the first closed position, and
the third tube is sandwiched between the wall and the second presser in a state where the second presser is located at the second closed position.

6. A clamp comprising one clamp provided in a branch in a blood bag system, wherein
the blood bag system includes a first bag configured to accommodate blood, a second bag configured to accommodate a blood component obtained by centrifuging the blood in the first bag, a third bag configured to accommodate an additive solution, a first tube connecting the first bag and the branch, a second tube connecting the branch and the second bag, and a third tube connecting the branch and the third bag,
the clamp includes
a first clamp mechanism configured to close a flow path of the second tube, and
a second clamp mechanism configured to close a flow path of the third tube,
the first clamp mechanism is configured not to open the flow path of the second tube that has been closed once, and
the second clamp mechanism is configured to open the flow path of the third tube that has been closed.

7. A blood bag system comprising:
a first bag configured to accommodate blood; a second bag configured to accommodate a blood component obtained by centrifuging the blood in the first bag; and a third bag configured to accommodate an additive solution, wherein
a first tube connected to the first bag is coupled, via a branch, to a second tube connected to the second bag and a third tube connected to the third bag,
the branch is provided with one clamp,
the clamp includes a first clamp mechanism and a second clamp mechanism,
the first clamp mechanism includes
a first arm having flexibility,
a first protrusion provided on the first arm and pressing an outer peripheral surface of the second tube to close a flow path of the second tube, and
a first engaging portion engaging with the first arm in a state where the flow path of the second tube is closed, and
the second clamp mechanism includes
a second arm having flexibility,
a second protrusion provided on the second arm and pressing an outer peripheral surface of the third tube to close a flow path of the third tube,
a second engaging portion engaging with the second arm in a state where the flow path of the third tube is closed, and
an engagement releaser releasing engagement between the second engaging portion and the second arm.

8. The blood bag system according to claim 7, wherein
the engagement releaser includes an operating portion protruding from a wall provided with the second engaging portion toward outside of the second arm.
